# EUROPEAN PATENT APPLICATION

(11) **EP 4 226 930 A1**
(43) Date of publication of application: **16.08.2023**
(21) Application number: 21892305.0
(22) Date of filing: 10.11.2021
(51) Int. Cl.: A61K 35/745, A61K 45/06, A61P 35/00, A23L 33/135, A23K 10/18

(54) **COMPOSITION FOR PREVENTING OR TREATING CANCER COMPRISING BIFIDOBACTERIUM LONGUM RAPO (KCTC13773BP)**

(30) Priority: 13.11.2020 KR 20200151618
(71) Applicant: Bifido Co., Ltd., Gangwon-do 25117 (KR)
(72) Inventor: PARK, Myeong Soo, Seoul 06091 (KR); JI, Geun Eog, Seoul 06715 (KR); KIM, Sung Eun, Seoul 04310 (KR); KIM, Hye Yoon, Seoul 04765 (KR); OH, Ri Ra, Seoul 08726 (KR); HEO, Ji Won, Ansan-si Gyeonggi-do 15483 (KR)
(74) Representative: Strych, Sebastian
(86) International application number: PCT/KR2021/016301
(87) International publication number: WO 2022/103138

(57) **Abstract**

The present invention relates to a composition for preventing or treating cancer comprising Bifidobacterium longum RAPO (KCTC13773BP), and when used together with immune checkpoint inhibitors (immune anticancer drugs), side effects of immune checkpoint inhibitors are reduced and the effect is more promoted, resulting in an excellent effect in preventing or treating cancer.

## Description

### [Technical Field]

The present invention relates to a composition for preventing or treating cancer containing *Bifidobacterium* longum RAPO (KCTC13773BP), and more specifically, to a composition for preventing or treating cancer that is highly effective in preventing or treating cancer by reducing the side effects of an immune checkpoint inhibitor while further improving the effects thereof to further activate the immune system, when used in combination with the immune checkpoint inhibitor (immuno-anticancer agent).

### [Background Art]

The number of cancer patients continues to increase all over the world. In Korea in 2016, 229,180 new cancer patients were diagnosed, which is an increase of 12,638 (5.8%) compared to the previous year. In particular, breast cancer is one of the five major cancer types and ranked first (for women) in cancer mortality as can be seen from the cause of death statistics in 2017 (Korea Central Cancer Registry, 2018) .

Starting with first-generation chemotherapeutic agents, targeted therapeutic agents were developed as second-generation anticancer drugs at the end of the 1990s. Since 2011, immuno-anticancer drugs have been developed, approved and actively used. In 2014, the U.S. Food and Drug Administration (FDA) approved pembrolizumab and nivolumab, anti-PD1 immune checkpoint inhibitors as therapeutic agents for metastatic melanoma, non-small cell lung cancer and various tumors, and the scope of application thereof is still widening. Then, anti-PDL1 immune checkpoint inhibitors such as atezolizumab, durvalumab, and avelumab were also approved by the FDA and the application thereof is expanding (Miwa S, Shirai T, Yamamoto N, et al. Current and emerging targets in immunotherapy for osteosarcoma. J Oncol 2019;2019:7035045).

Immune checkpoint inhibitors refer to immuno-anticancer agents that activate the immune system to attack cancer cells without directly killing cancer cells and are in the spotlight as next-generation anticancer drugs that are almost free of side effects caused by chemotherapy, such as hair loss, anemia, and suppression of bone marrow function, which reduce the quality of life of cancer patients, unlike conventional chemotherapy directly killing cancer cells.

However, recent clinical studies reported that there are patients (responders) who respond well to immune checkpoint inhibitors and patients (non-responders) who do not respond well to immune checkpoint inhibitors (Darvin P, Toor SM, Sasidharan Nair V, Elkord E. Immune checkpoint inhibitors: recent progress and potential biomarkers. Exp Mol Med. 2018;50(12):165), and especially a problem has been raised that the probability of patients responding to the drug is low when the immune checkpoint inhibitor is administered alone (Wei SC, et al,. Fundamental mechanisms of immune checkpoint blockade therapy. Cancer Discov.2018;8(9):1069-1086). Immune checkpoint inhibitors act as a mechanism to activate the immune system and thus are advantageously applicable to a large number of patients, unlike conventional chemotherapeutic or targeted therapeutic agents. However, immune checkpoint inhibitors may cause immune-related adverse events (irAEs) due to the imbalance of the immune system resulting from excessively increased T cell activity. Research has reported in 2019 that typical side effects of immune checkpoint inhibitors include colitis, pneumonia, hepatitis, myocarditis, thyroiditis, pituitary gland infection, nephritis, fatigue, diarrhea, and rash (Aratani Y et al., 2018; Baxi S et al., 2018; El Rayes T et al., 2015; Hong F et al., 2015). Therefore, the need for research on combination therapy to improve the therapeutic effect of immune checkpoint inhibitors and reduce side effects thereof is emerging.

It is known that homeostasis of intestinal microorganisms is maintained by continuous interaction with the body's immune system, and unbalance of these microorganisms (dysbiosis) may cause chronic diseases including cancer. Therefore, research in the field of pharmabiotics dealing with the influence of intestinal microbiota on the effect of anticancer therapy has recently attracted a great deal of attention (Cogdill AP et al. The impact of intratumoral and gastointestinal microbiota on systemic cancer therapy. Trends immunol. 2018;39(11) :900-920) .

In 2018, MD Anderson cancer center reported the effect of gut microbiome compositions on the effects of immune checkpoint inhibitors in melanoma. The intestinal microorganisms of responders and non-responders among melanoma patients receiving anti-PD1 immunotherapy were fed to germ-free mice through fecal microbiota transplantation (FMT). As a result, mice having received FMT from the responders exhibited an increase in CD8+ T cells and an increased response to anticancer drugs, resulting in a significant decrease in cancer growth (Gopalakrishnan V, Spencer CN, Nezi L, et al. Gut microbiome modulates response to anti-PD-1 immunotherapy in melanoma patients. Science. 2018;359(6371):97-103).

The research by the University of Chicago group published in Science in 2015 showed that, when mouse xenograft model derived from the melanoma cell line, B16.F10 cell line was treated with anti-PDL1 mAb, a higher amount of *Bifidobacterium* was found, especially in responders. When these *Bifidobacterium* were fed to non-responders, INF-γ and CD8+ T cells increased and cancer cell growth was suppressed, which supports that probiotics increase the activity of immune cells, thus contributing to the responsiveness of immune checkpoint inhibitors and the improvement of anticancer effects (Sivan A, Corrales L, Hubert N, et al. Commensal Bifidobacterium promotes antitumor immunity and facilitates anti-PD-L1 efficacy. Science. 2015;350(6264) :1084-1089).

### [Disclosure]

### [Technical Problem]

The present inventors found that the importance of studies on the effect of combination therapy of probiotics and immune checkpoint inhibitors, which have beneficial effects in the body such as strengthening immunity is emphasized in terms of pharmabiotics (pharmaceutical + probiotics). However, studies related to this in various carcinomas are still incomplete. Therefore, the present invention aims at determining the effect of the combination therapy of probiotics and anti-PD1 immune checkpoint inhibitors on the anticancer therapy effect.

### [Technical Solution]

In accordance with an aspect of the present invention, the above and other objects can be accomplished by the provision of a pharmaceutical composition for preventing or treating cancer containing *Bifidobacterium longum* RAPO (KCTC13773BP) .

In the pharmaceutical composition, the *Bifidobacterium longum* RAPO (KCTC13773BP) may exhibit an immune-enhancing effect as well as anticancer activity.

In the pharmaceutical composition, the cancer includes, for example, at least one selected from the group consisting of melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, hemangiosarcoma, mast cell tumor, leukemia, lymphoma, liver cancer, lung cancer, pancreatic cancer, stomach cancer, kidney cancer, colorectal cancer, hematopoietic tumor, neuroblastoma, epidermal carcinoma, and metastatic cancer thereof.

In accordance with another aspect of the present invention, provided is a pharmaceutical composition for preventing or treating cancer containing *Bifidobacterium longum* RAPO (KCTC13773BP) and further containing an immuno-anticancer agent.

In the pharmaceutical composition, the immuno-anticancer agent preferably includes at least one selected from the group consisting of anti-PD1, anti-PDL1, anti-CTLA, anti-Tim3 and anti-LAG3.

In the pharmaceutical composition, the *Bifidobacterium longum* RAPO (KCTC13773BP) strain and the immuno-anticancer agent are preferably administered simultaneously in one formulation, or administered simultaneously or sequentially in separate formulations.

In the pharmaceutical composition, the *Bifidobacterium longum* RAPO (KCTC13773BP) preferably reduces side effects of the immuno-anticancer agent.

In accordance with another aspect, provided is a food composition for preventing or ameliorating cancer containing *Bifidobacterium longum* RAPO (KCTC13773BP).

In accordance with another aspect, provided is an animal feed composition for preventing or ameliorating cancer containing *Bifidobacterium longum* RAPO (KCTC13773BP).

### [Advantageous effects]

In the present invention, it was confirmed that *Bifidobacterium longum* RAPO (KCTC13773BP) can be used as an anticancer agent. Moreover, when the *Bifidobacterium longum* RAPO (KCTC13773BP) of the present invention is used in combination with an immune checkpoint inhibitor (immuno-anticancer agent), the side effects of the immune checkpoint inhibitor are reduced and the effects thereof are further promoted, thereby providing potent effects of preventing or treating cancer.

### [Description of Drawings]

FIG. 1 illustrates weight change.
FIG. 2 illustrates the change in tumor volume.
FIG. 3 illustrates the concentrations of AST and ALT in serum.
FIG. 4 illustrates the concentrations of BUN and creatine in serum.
FIG. 5 illustrates the concentration of LDH in serum.
FIG. 6 is an IHC (PDL1) staining image (A) and IHC scores (B) of tumor tissues.
FIG. 7 shows the result of PDL1 mRNA expression in tumors.
FIG. 8 shows the result of flow cytometry analysis of CD4+ (A) and CD8+ (B) cells in the spleen and the CD8/CD4 ratio in CD3+ cells (C).
FIG. 9 shows the result of flow cytometry analysis of NKT cells in the spleen.
FIG. 10 shows the result of flow cytometry analysis of NK cells in the spleen.
FIG. 11 shows the results of flow cytometry analysis of the DCs (A), MDSCs (B), and macrophages (C) in CD3+ cells in the spleen.
FIG. 12 shows results of flow cytometry analysis of CD4+ (A) and CD8+ (B) in CD3+ cells in tumors, and CD8/CD4 ratio (C) in CD3+ cells.
FIG. 13 shows the result of flow cytometry analysis of NK cells in tumors.
FIG. 14 shows the result of flow cytometry analysis of DC cells in tumors.
FIG. 15 shows the results of flow cytometry analysis of M1 (A) and M2 (B) in tumors and the M2/M1 ratio (C).
FIG. 16 shows the result of IFNγ mRNA expression in tumors.
FIG. 17 shows the result of TNFα mRNA expression in tumors.
FIG. 18 shows the result of CXCL9 mRNA expression in tumors.
FIG. 19 shows the result of CCL2 mRNA expression in tumors.
FIG. 20 shows the result of mRNA expression of IL10 (A), TGFβ (B) and Arg1 (C) in tumors.
FIG. 21 shows the result of HMGB1 mRNA expression in tumors.
FIG. 22 shows the result of CRT mRNA expression in tumors.
FIG. 23 shows the result of CD80 mRNA expression in tumors.
FIG. 24 shows the result of CD86 mRNA expression in tumors.
FIG. 25 shows the results of intratumoral IL1β (A), IL12 (B) and Gzmb (C) mRNA expression.
FIG. 26 shows the summary of the molecular mechanisms involved in the effect of combination of the *Bifidobacterium longum* RAPO strain of the present invention and immune checkpoint inhibitors in a mouse xenograft model.

### [Best Mode]

The present invention provides a pharmaceutical composition for preventing or treating cancer containing a *Bifidobacterium longum* RAPO (KCTC13773BP) strain. The *Bifidobacterium longum* RAPO (KCTC13773BP) strain of the present invention has been registered in Korean Patent No. 10-2074445 (registration date: 2020. 01. 13).

The *Bifidobacterium longum* RAPO (KCTC13773BP) strain of the present invention may include the strain itself, a culture solution of the strain, or a cytoplasmic fraction obtained by disrupting the strain.

The present invention provides a method for preventing or treating cancer including administering an effective amount of *Bifidobacterium longum* RAPO (KCTC13773BP) strain to a patient or to a human or non-human animal in need of cancer prevention or treatment. The non-human animal includes all vertebrates, such as mammals.

The *Bifidobacterium longum* RAPO (KCTC13773BP) strain of the present invention exhibits an immunity-enhancing effect as well as anticancer effect.

In the present invention, the cancer includes, but is not limited to, melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, hemangiosarcoma, mast cell tumor, leukemia, lymphoma, liver cancer, lung cancer, pancreatic cancer, stomach cancer, kidney cancer, colorectal cancer, hematopoietic tumor, neuroblastoma, epidermal carcinoma, and metastatic cancer thereof.

The present invention provides a pharmaceutical composition for preventing or treating cancer containing *Bifidobacterium longum* RAPO (KCTC13773BP) and further containing an immuno-anticancer agent. That is, the present invention includes the *Bifidobacterium longum* RAPO (KCTC13773BP) strain, and may further include an immuno-anticancer agent. The *Bifidobacterium longum* RAPO (KCTC13773BP) strain and the immuno-anticancer agent are preferably administered simultaneously in one formulation, or are administered simultaneously or sequentially in separate formulations. The immuno-anticancer agent may, for example, include anti-PD1, anti-PDL1, anti-CTLA, anti-Tim3, or antiLAG3 acting as an immune checkpoint inhibitor, but is not limited thereto. In the pharmaceutical composition for preventing or treating cancer of the present invention, the *Bifidobacterium longum* RAPO (KCTC13773BP) preferably reduces the side effects of the immuno-anticancer agent.

The present invention provides a food composition or animal feed composition for preventing or ameliorating cancer containing a *Bifidobacterium longum* RAPO (KCTC13773BP) strain. The food composition may be, for example, health functional food, dairy product, or the like, but is not necessarily limited thereto. In addition, the feed composition may be prepared by various methods widely known in the art, such as pelletization, but is not necessarily limited to a specific formulation. A detailed description of this formulation will be omitted.

Meanwhile, immune checkpoint inhibitors are next-generation anticancer drugs that exhibit anticancer effects by strengthening the body's immune system without directly killing cancer cells and can be applied to various types of cancer, but have a drawback in that there are responders and non-responders. Therefore, the need for research on combination therapy to enhance the therapeutic effect of these immune checkpoint inhibitors has emerged and it has recently been reported that the composition of intestinal microorganisms is related to the treatment of immune checkpoint inhibitors.

Accordingly, in the present invention, the mouse breast cancer model was administered a single immune checkpoint inhibitor and was subjected to a combination therapy of the immune checkpoint inhibitor and *Bifidobacterium* to identify the effect of combination therapy on enhancement of the anti-cancer treatment of the immune checkpoint inhibitor and related molecular mechanisms, and to discover biomarkers. The following effects of the present invention were experimentally proved from the following experiments of the present invention.

### (1) Effect of combination therapy of immune checkpoint inhibitor and Bifidobacterium of present invention on tumor size

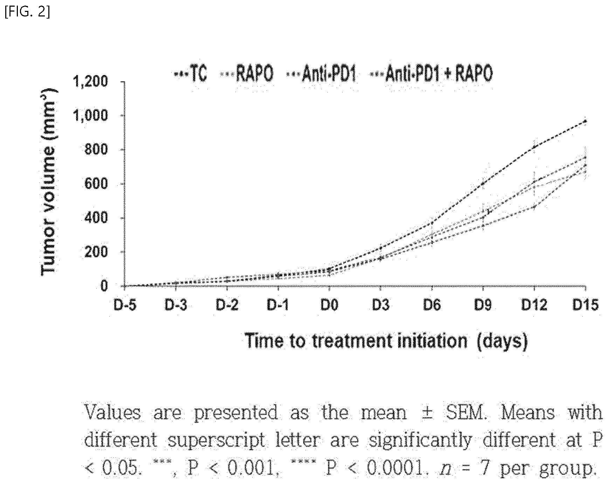

The group treated with a combination of anti-PD1 as an immune checkpoint inhibitor and *Bifidobacterium longum* RAPO as *Bifidobacterium* exhibits the lowest tumor volume during therapy (FIG. 2), and exhibited greater carcass weight than the group treated with anti-PD1 alone (Table 3). This supports that the combination therapy of the immune checkpoint inhibitor with *Bifidobacterium longum* RAPO is capable of enhancing anti-cancer treatment of the immune checkpoint inhibitor.

### (2) Effect of combination therapy of immune checkpoint inhibitor and Bifidobacterium of present invention on reducing side effects

The tissue weight was compared between respective groups. The result showed that the Anti-PD1 group had increased liver weight compared to the other groups, while the Anti-PD1 + RAPO group had decreased liver weight compared to the Anti-PD1 group (Table 4). It has been reported that an increase in liver weight indicates abnormal liver metabolism, fibrosis, and inflammation (Lee JE, Kang SJ, Choi SH, Song CH, Lee YJ, Ku SK. Fermentation of Green Tea with 2% Aquilariae lignum Increases the Anti-Diabetic Activity of Green Tea Aqueous Extracts in the High Fat-Fed Mouse. Nutrients. 2015;7(11):9046-9078. Published 2015 Nov 3. doi:10.3390/nu7115447), which supports that combination therapy of the immune checkpoint inhibitor and *Bifidobacterium* is capable of alleviating hepatomegaly that may be caused by the immune checkpoint inhibitor. In addition, it was confirmed that the colon length of the Anti-PD1 + RAPO group was significantly greater than that of the Anti-PD1 group (Table 6). A decrease in colon length is a typical feature observed in the colitis mouse model. The results of this study show that the risk of colitis increases as a side effect of the treatment with the immune checkpoint inhibitor, and the combination therapy of the immune checkpoint inhibitor and *Bifidobacterium* is capable of mitigating side effects that may be caused by the immune checkpoint inhibitor administration.

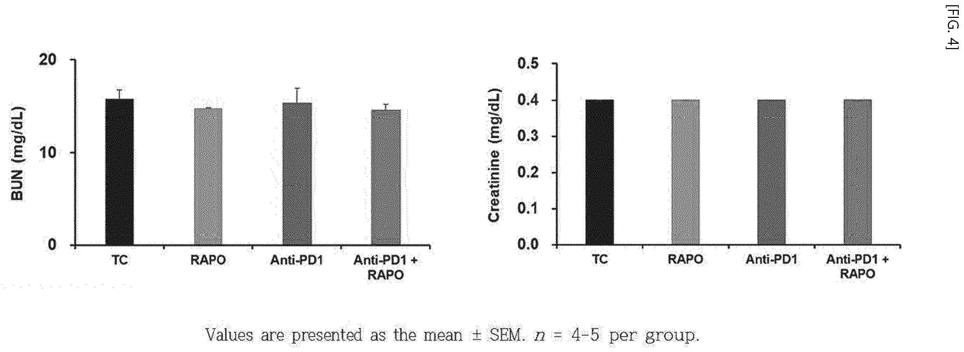

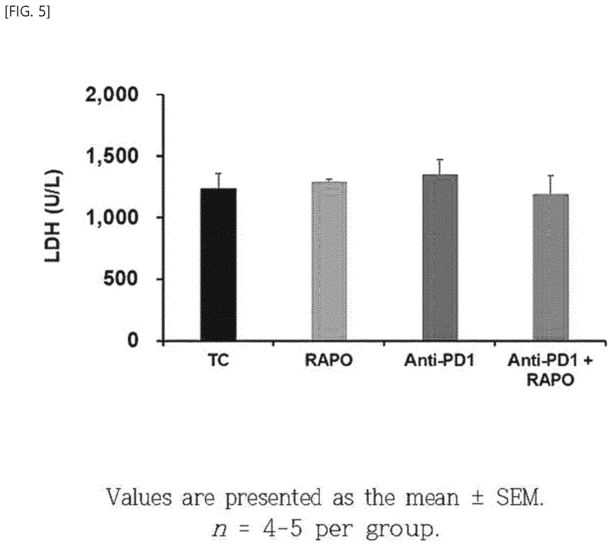

In addition, in order to determine the effect of mitigating side effects by the combined treatment of the immune checkpoint inhibitor and *Bifidobacterium,* various biochemical indicators were detected in the serum of each group. As a result, the concentration of BUN decreased in the Anti-PD1 + RAP group compared to the Anti-PD1 group (Table 8, FIG. 4), and the concentration of LDH in the blood increased in the Anti-PD1 group, but decreased in the Anti-PD1 + RAPO group (Table 9, FIG. 5). It is reported that 16-37% of patients treated with anti-PD1 drugs, among immune checkpoint inhibitors, feel fatigue due to the immune checkpoint inhibitors. Although such fatigue is simply regarded as one of the side effects, it is known to be related to the occurrence of side effects associated with the endocrine glands. Therefore, whether or not a larger side effect occurred could be determined by measurement of the LDH concentration in the blood (Naidoo J, Page DB, Li BT, et al. Toxicities of the anti-PD-1 and anti-PD-L1 immune checkpoint antibodies [published correction appears in Ann Oncol. 2016 Jul;27(7):1362]. Ann Oncol. 2015;26(12):2375-2391. doi:10.1093/annonc/mdv383). In addition, as the ability of the kidney to remove urea decreases, the concentration of BUN in blood increases (Edelstein C. L. (2008). Biomarkers of acute kidney injury. Advances in chronic kidney disease, 15(3), 222-234 https://doi.org/10.1053/j.ackd.2008.04.003). For this reason, the results of this experiment showed that ingestion of *Bifidobacterium* at the same time as administration of immune checkpoint inhibitors can relieve deterioration of kidney function and fatigue caused by immune checkpoint inhibitors.

### (3) Effect of combination therapy of immune checkpoint inhibitor and Bifidobacterium of present invention on PDL1 expression in tumor tissue

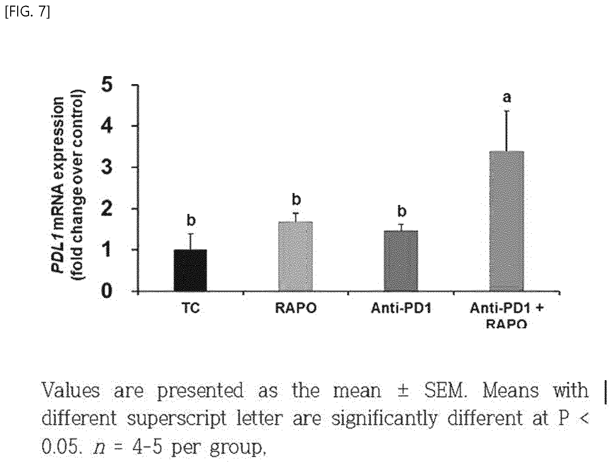

The difference in PDL1 expression in the tumor tissue between respective groups was detected through IHC and qRT-PCR. The result showed that the Anti-PD1 + RAPO group exhibited significantly increased PDL1 expression compared to the other three groups (FIG. 7). Research showed that the level of PDL1 can be used as a biomarker to directly determine the response to PD1/PDL1 therapy and PDL1 is highly expressed in cancer cells of patients with high progression-free survival and overall survival, among non-small cell lung cancer patients treated with immune checkpoint inhibitors. In addition, overexpression of PDL1 in tumors is known to be associated with tumor-infiltrating lymphocytes that secrete IFNγ (Darvin P, Toor SM, Sasidharan Nair V, Elkord E. Immune checkpoint inhibitors: recent progress and potential biomarkers. Exp Mol Med. 2018 Dec 13;50(12) :1-11). Therefore, IHC and qRT-PCR results indicate that the combination therapy of immune checkpoint inhibitors and *Bifidobacterium longum* RAPO is capable of increase the responsiveness of immune checkpoint inhibitors by improving the expression of PDL1 in tumors.

### (4) Effects of combination therapy of immune checkpoint inhibitor and Bifidobacterium of present invention on immune cell activity

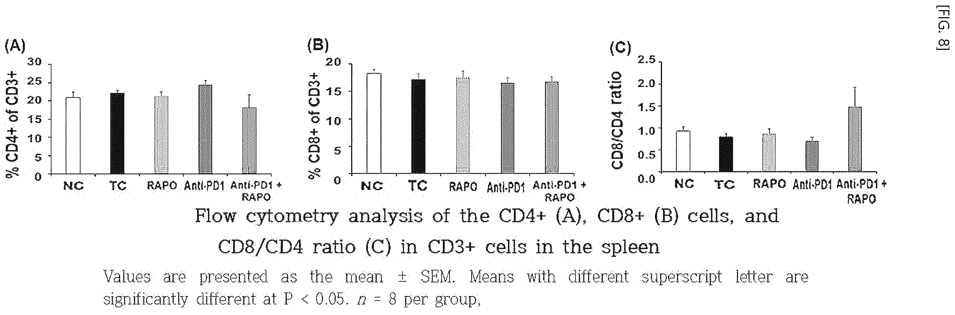

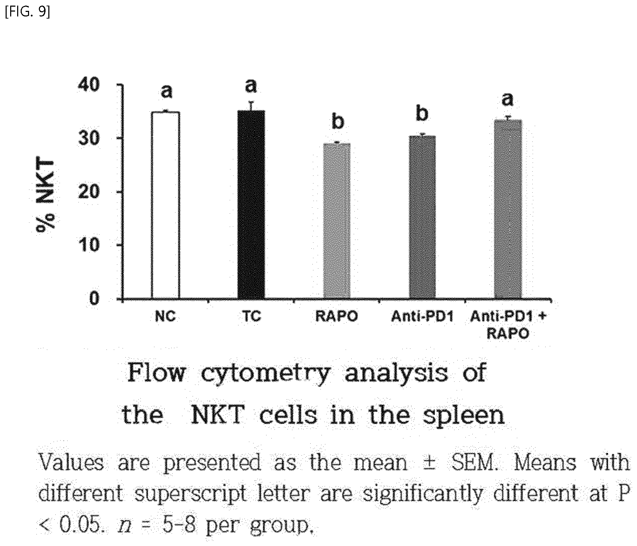

Flow cytometry experiments were conducted on spleen and tumor tissues to identify the activation of immune cells during the combination therapy of immune checkpoint inhibitors and *Bifidobacterium.* The CD8/CD4 ratio in the spleen of the Anti-PD1 + RAPO group increased compared to the other groups (C of FIG. 8) . CD8 T cells with cytotoxicity are known to play a direct role in anti-cancer therapy using anti-PD1 in response to tumor-specific antigens in relation to tumor suppression (Leclerc M, Voilin E, Gros G, Corgnac S, de Montpreville V, Validire P, Bismuth G, Mami-Chouaib F. Regulation of antitumour CD8 T-cell immunity and checkpoint blockade immunotherapy by Neuropilin-1. Nat Commun. 2019 Jul 26;10(1) :3345). Therefore, the increase of the CD8/CD4 ratio was used to identify the possibility of enhancing the therapeutic effect of anti-PD1 in the combination therapy of the immune checkpoint inhibitor and *Bifidobacterium.* In addition, the activity of NKT cells is known to function to increase antitumor immunity by reactivating exhausted CD8 T cells (Bae EA, Seo H, Kim BS, Choi J, Jeon I, Shin KS, Koh CH, Song B, Kim IK, Min BS, Han YD, Shin SJ, Kang CY. Activation of NKT Cells in an Anti-PD-1-Resistant Tumor Model Enhances Antitumor Immunity by Reinvigorating Exhausted CD8 T Cells. Cancer Res. 2018 Sep 15;78(18) :5315-5326). It was confirmed that NKT cells increased significantly in the Anti-PD1 + RAPO group compared to the Anti-PD1 group (FIG. 9), which indicates that the combination therapy of *Bifidobacterium* with the immune checkpoint inhibitor improved the therapeutic effect of the immune checkpoint inhibitor through the activity of immune cells such as NKT cells in the spleen tissue.

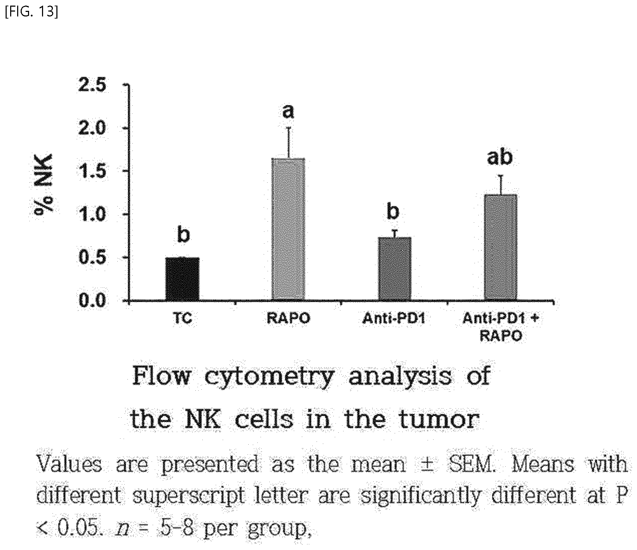

It can be seen from flow cytometry experiments in the tumor tissue that the Anti-PD1 + RAPO group slightly increased the number of NK cells compared to the Anti-PD1 group (FIG. 13). It is known that NK cells in the tumor microenvironment are closely related to positive treatment prognosis in various carcinomas and that they have the ability to directly attack tumor cells and primarily inhibit the metastasis of tumor cells (Larsen SK, Gao Y, Basse PH. NK cells in the tumor microenvironment. Crit Rev Oncog. 2014;19(1-2):91-105. doi:10.1615/critrevoncog.2014011142). Therefore, the combination therapy of anti-PD1 and *Bifidobacterium longum* RAPO can maximize the therapeutic effect of the immune checkpoint inhibitor based on the increase in the number of NK cells that have anti-tumor functions in tumor tissue.

### (5) Effect of combination therapy of immune checkpoint inhibitors and Bifidobacterium on tumor-associated macrophages (TAM)

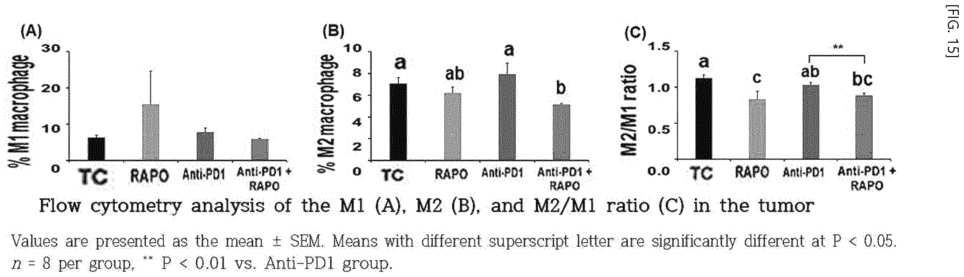

Recently, studies on the resistance of immune checkpoint inhibitors have been actively conducted, and in particular, the possibility that the resistance of immune checkpoint inhibitors may be related to tumor-associated myeloid cell infiltrates such as tumor-associated macrophages has been raised (Pathria P, Louis TL, Varner JA. Targeting tumor-associated macrophages in cancer. Trends Immunol.2019;40(4) :310-327). Tumor-associated macrophages (TAMs) are largely differentiated into two types, namely, M1 and M2. The former plays roles in antigen presentation and pro-inflammatory cytokine secretion, while the latter is a macrophage that plays a role in anti-inflammatory cytokine secretion and immunosuppression. That is, it is known that M1 acts as an anti-tumor and M2 acts as a pro-tumor in a tumor environment (Lu D, Ni Z, et al. Beyond T cells: understanding the role of PD-1/PD-L1 in tumor-associated macrophages. J Immunol Res. 2019;2019:1919082. Huang Y, Snuderl M, Jain RK. Polarization of tumor-associated macrophages: a novel strategy for vascular normalization and antitumor immunity. Cancer cell. 2011;19(1) :1-2). The result of the study of the present invention showed that the ratio of M2 macrophages and M2/M1 macrophages in tumor tissue significantly decreased in the Anti-PD1 + RAPO group compared to the Anti-PD1 group (FIG. 15) .

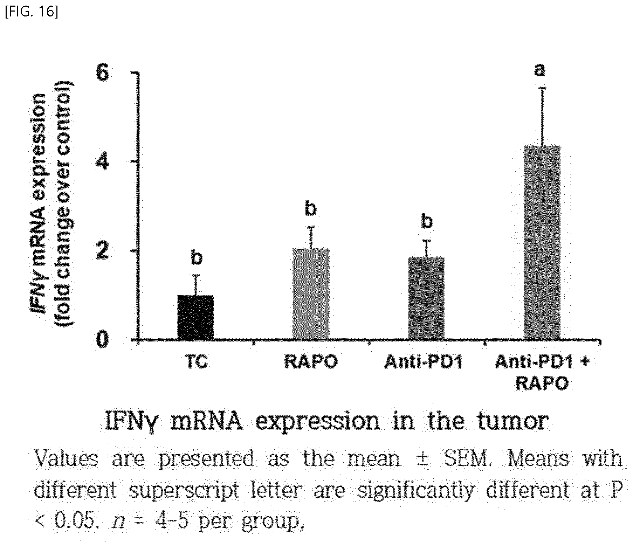

In addition, TAM-related markers were detected in tumor tissue through qRT-PCR. The result showed that, compared to the Anti-PD1 group, the Anti-PD1 + RAPO group exhibited a significant increase in the gene expression of IFNγ, TNFα, CXCL9, and IL1β associated with M1 macrophages, and exhibited a significant decrease in IL10 and Arg1 associated with M2 macrophages, and a decrease in TGFβ and CCL2 (FIGS. 16 to 20). It can be seen from these results that the combination therapy of immune checkpoint inhibitors and *Bifidobacterium* could enhance the anticancer treatment effect by which the anti-tumor pathway of TAMs are activated and the pro-tumor pathway thereof are suppressed.

### (6) Effects of combination therapy of immune checkpoint inhibitor and Bifidobacterium on immunogenic cell death (ICD)

In the tumor microenvironment, the ICD is known to play a role in inducing the anti-tumor immune system. It is known that receptors and ligands on the surface of dendritic cells (DCs) are attracted through DAMPs secreted from dead cancer cells and are converted into mature phenotypes, to increase phagocytosis to antigens of DCs and cancer cell death and facilitate anti-tumor activity (Zhou J, Wang G, et al. Immunogenic cell death in cancer therapy: present and emerging inducers. J cell mol med. 2019;23(8) :4854-4865) .

It is known that the ICD plays an important role in chemotherapy, radiotherapy, and anticancer treatment using immune checkpoint inhibitors and that the composition of intestinal microbiomes affects the ICD process (Chen W, Wang S, Wu Y, Shen X, Guo Z, Li Q, Xing D. Immunogenic cell death: A link between gut microbiota and anticancer effects. Microb Pathog. 2020 Apr;141:103983.). The research published in Nature Communications in 2018 showed that CP1, which is a prostate-specific microorganism derived from a patient, improved the anti-cancer effect of immune checkpoint inhibitors using anti-PD1 and increased survival through ICD activity of cancer cells (Anker JF, Naseem AF, Mok H, Schaeffer AJ, Abdulkadir SA, Thumbikat P. Multi-faceted immunomodulatory and tissue-tropic clinical bacterial isolate potentiates prostate cancer immunotherapy. Nat Commun. 2018 Apr 23;9(1):1591). As such, it was considered that intestinal microorganisms affect ICD through immune responses and are ultimately closely related to the reactivity of immune checkpoint inhibitors.

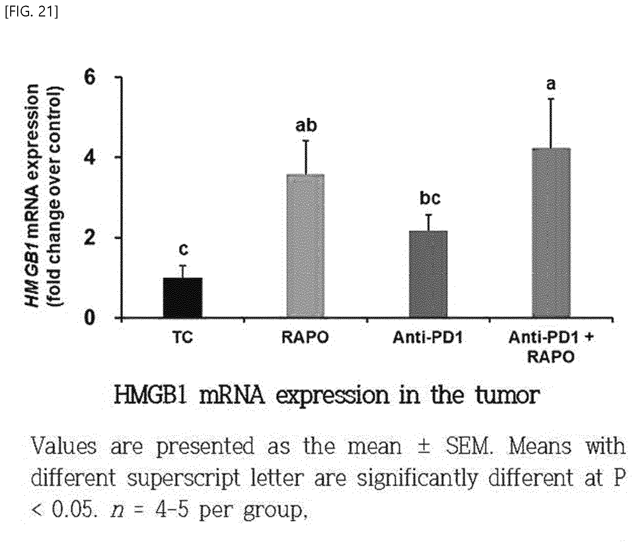

In the present invention, the gene expression of ICD-related markers in tumor tissue was identified through qRT-PCR. The result showed that the gene expression of HMGB1 and CRT, as DAMPs, increased significantly in the Anti-PD1 + RAPO group compared to the Anti-PD1 group. In addition, during the combination therapy, the expression of IL1β, IL12, and IFNγ, which are secreted by the activity of ICD and CD80, which are markers of mature DCs, increased significantly, and the expression of Gzmb increased (FIGS. 21 to 25). The research results indicate that the combination therapy of *Bifidobacterium longum* RAPO with an immune checkpoint inhibitor activates the ICD, thereby further activating anti-tumor immunity.

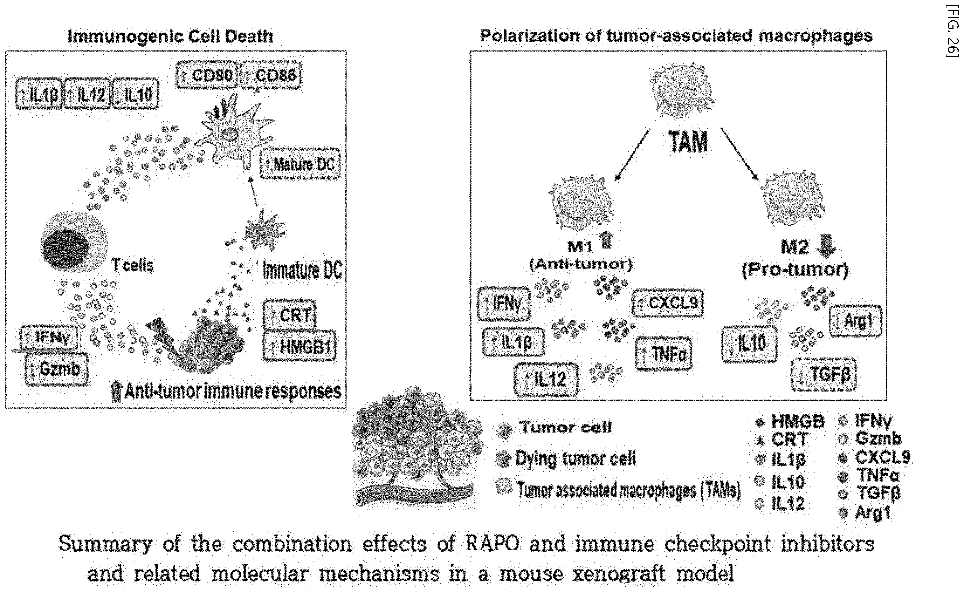

FIG. 26 shows the summary of the molecular mechanisms involved in the effects of combination of the *Bifidobacterium longum* RAPO strain of the present invention and immune checkpoint inhibitors in a mouse xenograft model.

Hereinafter, the present invention will be described in more detail with reference to the following examples and experimental examples, but the scope of the present invention is not limited to the examples and experimental examples, and includes variations and technical concepts equivalent thereto.

### [Example 1: Confirmation of enhancement of therapeutic effect of anti-PD1 immune checkpoint inhibitor by Bifidobacterium of present invention]

### 1. Experiment method

### A. Laboratory animal

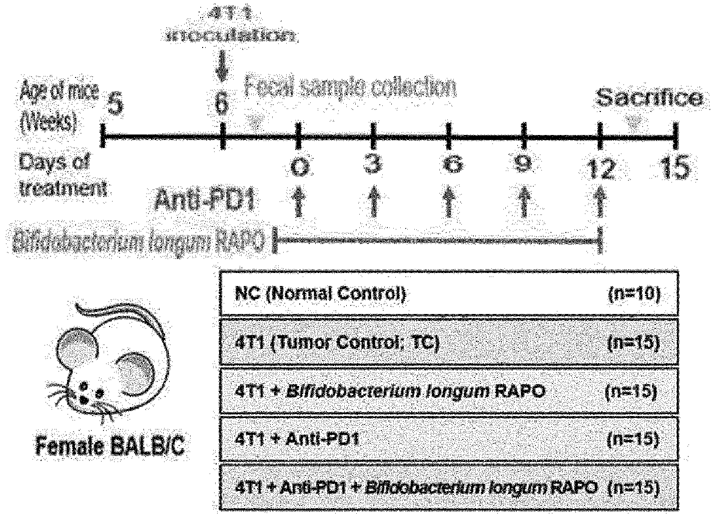

5-week-old female BALB/c mice were purchased from Central Lab Animal Inc. (Seoul, Korea) and fed chow diet and water in a 12/12 hour light/dark cycle at a temperature of 22 ± 1°C. After a one-week adaptation period, the mice were fed AIN-76A diet, and a 4T1 mouse breast cancer cell line was subcutaneously injected into the mice to induce a breast cancer animal model and then randomly selected so that the average weight per group was similar, and classified into 5 groups as follows: ① Normal Control (NC), ② Tumor Control (TC), ③ Group administered Bifidobacterium only (*Bifidobacterium longum* RAPO; RAPO), ④ Group administered Immune checkpoint inhibitor (Anti-PD1), ⑤ Combination treatment group (Anti-PD1 + *Bifidobacterium longum* RAPO; Anti-PD1 + RAPO).

When the tumor volume reached 50-150 mm³ after breast cancer cell line administration, the Anti-PD1 group and the Anti-PD1 + RAPO group were fed a solution of the immune checkpoint inhibitor Anti-PD1 (Bioxcell) in phosphate buffered saline (PBS) once every 3 days by intraperitoneal injection at 200 µg/mouse and *Bifidobacterium longum* RAPO were orally administered to the RAPO group and the Anti-PD1 + RAPO group daily from 2 days before the first dose of Anti-PD1 (1×10⁹ CFU/mouse). During the experimental period, body weight, food intake, and tumor size were measured two or three times a week at predetermined times. An anesthetic was inhaled to prevent movement of the experimental animals and to minimize pain and euthanasia was performed through cardiac blood sampling. Blood was collected from the heart and centrifuged at 3,000 rpm and 4°C for 20 minutes to isolate serum. Tissues were removed, washed with saline, dried, and weighed. A portion of the spleen and tumor tissue for flow cytometry was cut and injected into Dulbecco's Modified Eagle Medium (DMEM) on ice, and the remaining tissue was rapidly frozen in liquid nitrogen and stored in a -80°C deep freezer.

### B. Measurement of serum biochemical parameter

Indicators were analyzed from the serum isolated from mouse blood using AU480 (Beckman Coulter, USA). Aspartate aminotransferse (AST), alanine aminotransferase (ALT) and lactate dehydrogenase (LDH) were tested in accordance with the standard method of the international federation of clinical chemistry (IFCC). Blood urea nitrogen (BUN) was analyzed using an enzymatic method using urease, and creatinine was analyzed using the Jaffe method.

### C. Observation of PD-L1 expression in tumor tissue through immunohistochemistry (IHC) staining

A part of the tumor tissue was washed with saline, fixed in 10% formalin to produce paraffin sections, and IHC staining was performed using PD-L1 antibody (eBioscience). The stained tumor nodules were observed under an optical microscope (Aperio Sanscope AT, Aperio ImageScope (Leica Biosystems, Buffalo Grove, IL, USA), and the level of PD-L1 expression was detected.

### D. Flow cytometry of tumor and spleen tissue

Predetermined amounts of tumor tissue and spleen obtained from mice were added to DMEM and filtered using a cell strainer and a 1 ml syringe. Cells obtained through this process were treated with RBC lysis buffer and washed twice with PBS. The cells were mixed with FACS staining buffer, mixed with CD3, CD4, CD8a, CD45, CD11b, F4/80, CD206, CD38, NKp46, Gr1, CD103, and I-A/I-E antibodies, and allowed to stand at 4°C for 30 minutes, followed by FACS analysis.

### E. Comparison of immune-related gene expression through RT-qPCR analysis of tumor tissue

Trizol solution was added to a predetermined amount of tumor tissue to perform homogenization and then chloroform was added thereto to perform centrifugation. 70% DEPC ethanol was added to the collected pellet, followed by centrifugation to separate the pellet. The pellet was air-dried and then DEPC was added thereto to confirm the purity of RNA and was quantified at 0.25 pg/ul. After quantification, cDNA was synthesized using a cDNA synthesis kit (Genepole, Seoul, Korea). cDNA, primer, and SYBR kit (Genepole, Seoul, Korea) were mixed and RT-qPCR was performed using a 7500 ABI real-time PCR system.

### 2. Experimental results

### A. Weight, dietary intake and tumor volume

### (1) Comparison in weight change and final weight between groups

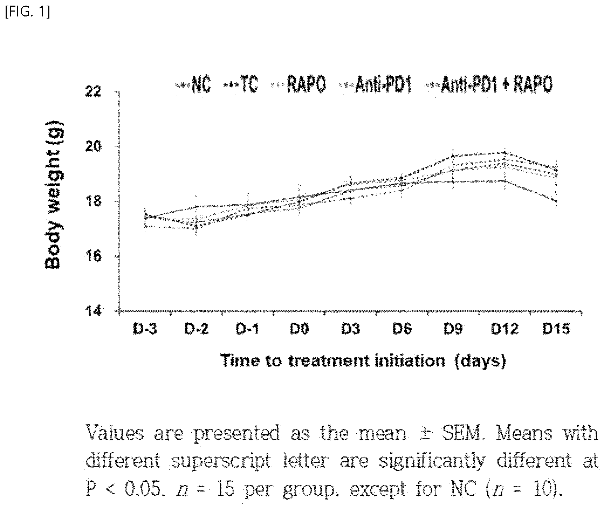

Weight change and weight immediately before sacrifice were compared between Normal Control (NC), Tumor Control (TC), Group administered Bifidobacterium only (*Bifidobacterium longum* RAPO; RAPO), Group administered Immune checkpoint inhibitor (Anti-PD1), and Combination treatment group (Anti-PD1 + *Bifidobacterium longum* RAPO; Anti-PD1 + RAPO) and the results are shown in FIG. 1 and Table 1, respectively. On day 15, the last of the 15 days after inoculation of breast cancer cell 4T1 and then administration of the immune checkpoint inhibitor, the weight of the NC group was significantly lower than that of the other four groups (P=0.0385) (FIG. 1, Table 1).

**[Table 1] Final weight**

| Group | Body weight (g) |
|---|---|
| NC | 18.02±0.32^{b} |
| TC | 19.13±0.17^{a} |
| RAPO | 18.83±0.25^{a} |
| Anti-PD1 | 19.24±0.27^{a} |
| Anti-PD1 + RAPO | 18.97±0.26^{a} |
| P-value | 0.0385 |

| | |
|---|---|
| Note) Values are presented as mean ± SEM. Means with different superscript letters are significantly different at P < 0.05. n = 15 per group, except for NC (n = 10). Note) Standard error of mean (SEM) | |

### (2) Dietary intake

Table 2 shows the mean food intake for each group. All groups were fed the AIN-76A diet and the difference in food intake therebetween was observed. The result showed that the food intake of the Anti-PD1 + RAPO group was significantly decreased compared to each of the other four groups, and the NC group had a significant decrease in food intake compared to the TC group (P<.0001).

**[Table 2] Dietary intake**

| Group | Food intake (g/day) |
|---|---|
| NC | 2.22±0.01^{b} |
| TC | 2.31±0.02^{a} |
| RAPO | 2.27±0.01^{ab} |
| Anti-PD1 | 2.27±0.02^{ab} |
| Anti-PD1 + RAPO | 2.15±0.02^{c} |
| P-value | <.0001 |

| | |
|---|---|
| Note) Values are presented as mean ± SEM. Means with different superscript letters are significantly different at P < 0.05. n = 15 per group, except for NC (n = 10). | |

### (3) Tumor volume change

FIG. 2 shows the tumor volume change of the 4T1 xenograft model of this experiment. The tumor volume was measured based on the day anti-PD1 was first administered. The result showed that, on Day 9, the tumor volume increased significantly in the TC group compared to the other three groups (P=0.0005), and on Day 12, the tumor volume of the Anti-PD1 + RAPO group decreased significantly compared to the TC and Anti-PD1 groups, the Anti-PD1 + RAPO group had the lowest mean of the tumor volume among the four groups and the tumor volume of the RAPO group decreased significantly compared to the TC group (P<.0001). On Day 15, the tumor volume of all three other groups significantly decreased compared to the TC group (P=0.0005) (FIG. 2).

### B. Comparison of tissue weight after dissection

Table 3 shows the results of comparison in carcass weight (weight of carcass excluding tumor after animal sacrifice) and cancer weight between the four groups injected with cancer cells. There was no significant difference in carcass weight between the groups, but the RAPO group and the Anti-PD1 + RAPO group administered *Bifidobacterium longum* RAPO exhibited an increase in carcass weight. There was no significant difference in cancer weight (Table 3).

**[Table 3] Carcass and tumor weights**

| Group | Carcass (g) | Tumor (g) |
|---|---|---|
| TC | 18.29±0.42 | 0.56±0.05 |
| RAPO | 18.75±0.32 | 0.45±0.06 |
| Anti-PD1 | 18.13±0.24 | 0.48±0.02 |
| Anti-PD1 + RAPO | 18.71±0.29 | 0.55±0.05 |
| P-value | 0.4742 | 0.3049 |

| | | |
|---|---|---|
| Note) Values are presented as mean ± SEM. n = 7 per group. | | |

Table 4 shows the weights of brain, lung, liver and kidney of respective groups. There was no significant difference in brain and kidney weights between all groups. All four groups injected with cancer cells exhibited a significant increase in weights of the lung and liver compared to the NC group (lung, P=0.0443; liver, P<.0001). The Anti-PD1 group exhibited an increase in liver weight compared to other groups, whereas the Anti-PD1 + RAPO group decreased compared to the Anti-PD1 group (Table 4).

**[Table 4] Brain, lung, liver and kidney weights**

| Group | Brain (g) | Lung (g) | Liver (g) | Kidney (g) |
|---|---|---|---|---|
| NC | 0.40±0.00 | 0.13±0.00^{b} | 0.69±0.01^{b} | 0.2510.00 |
| TC | 0.43±0.03 | 0.16±0.00^{a} | 0.85±0.02^{a} | 0.2610.00 |
| RAPO | 0.39±0.01 | 0.16±0.00^{a} | 0.85±0.04^{a} | 0.27±0.01 |
| Anti-PD1 | 0.41±0.01 | 0.16±0.01^{a} | 0.94±0.04^{a} | 0.2610.01 |
| Anti-PD1 + RAPO | 0.41±0.01 | 0.16±0.01^{a} | 0.89±0.03^{a} | 0.2610.01 |
| P-value | 0.4605 | 0.0443 | <.0001 | 0.6312 |

| | | | | |
|---|---|---|---|---|
| Note) Values are presented as mean ± SEM. Means with different superscript letters are significantly different at P < 0.05. n = 7 per group. | | | | |

Table 5 shows the heart, spleen and thymus weights of respective groups. There was no significant difference between the groups in the weights of the heart and thymus, whereas the other four groups exhibited a significant increase in the weight of the spleen compared to the NC group (P<.0001) (Table 5).

**[Table 5] Heart, spleen and thymus weights**

| Group | Heart (g) | Spleen (g) | Thymus (g) |
|---|---|---|---|
| NC | 0.09±0.00 | 0.09±0.00^{b} | 0.05±0.00 |
| TC | 0.10±0.00 | 0.66±0.03^{a} | 0.05±0.00 |
| RAPO | 0.10±0.00 | 0.57±0.03^{a} | 0.05±0.00 |
| Anti-PD1 | 0.09±0.00 | 0.65±0.06^{a} | 0.04±0.00 |
| Anti-PD1 + RAPO | 0.09±0.00 | 0.65±0.03^{a} | 0.05±0.00 |
| P-value | 0.4968 | <.0001 | 0.3459 |

| | | | |
|---|---|---|---|
| Note) Values are presented as mean ± SEM. Means with different superscript letter are significantly different at P < 0.05. n = 7 per group. | | | |

Table 6 shows the weights of the small and large intestines and the length of the large intestine for the five groups. There was no significant difference between the groups in the weight of the small intestine and the weight of the large intestine, but the Anti-PD1 group had a significantly decreased length of the large intestine, whereas the Anti-PD1 + RAPO group had an increased length of the large intestine than that of the Anti-PD1 group (P=0.0151) (Table 6).

**[Table 6] Small intestine weight, large intestine weight and large intestine length**

| Group | Small intestine weights (g) | Colon weights (g) | Colon length (cm) |
|---|---|---|---|
| NC | 0.80±0.03 | 0.16±0.01 | 8.97±0.16^{a} |
| TC | 0.84±0.04 | 0.13±0.01 | 8.89±0.24^{a} |
| RAPO | 0.87±0.03 | 0.13±0.01 | 8.16±0.39^{ab} |
| Anti-PD1 | 0.84±0.02 | 0.13±0.01 | 7.6610.30^{b} |
| Anti-PD1 + RAPO | 0.78±0.02 | 0.14±0.01 | 8.60±0.27^{a} |
| P-value | 0.2776 | 0.1482 | 0.0151 |

| | | | |
|---|---|---|---|
| Note) Values are presented as mean ± SEM. Means with different superscript letters are significantly different at P < 0.05. n = 7 per group. | | | |

### C. Measurement of serum biochemical parameters

The results of previous colon length measurement show that combination therapy with immune checkpoint inhibitors and *Bifidobacterium* is capable of mitigating various side effects that may be caused by immuno-anticancer drugs. Based thereon, to further investigate this, the concentrations of aspartate aminotransferse (AST), alanine aminotransferase (ALT), blood urea nitrogen (BUN), creatinine, and lactate dehydrogenase (LDH) in the serum of experimental animals were measured.

### (1) Liver function indicators (AST, ALT)

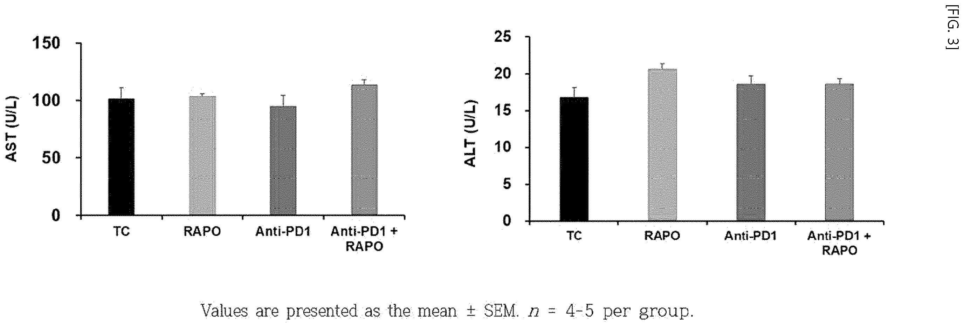

Concentrations of AST and ALT, which may be used to detect liver dysfunction, are shown in Table 7 and FIG. 3, respectively. AST and ALT are blood enzyme biomarkers that can determine the degree of damage to the liver. When inflammation occurs, hepatocytes are destroyed, and AST and ALT present in the liver are released therefrom and exist in the blood. Therefore, as the serum AST and ALT increases, damage to the liver increases. In particular, more ALT is present in the liver than in other tissues. Therefore, as the concentration of ALT in the blood increases, damage to the liver increases (Toita, R., Kawano, T., Fujita, S., Murata, M., & Kang, J. H. (2018). Increased hepatic inflammation in a normal-weight mouse after long-term high-fat diet feeding. Journal of toxicologic pathology, 31(1), 43-47) .

The result of this experiment showed that there was no significant difference in the concentrations of AST and ALT in the blood between the four groups, but the concentration of AST in blood decreased slightly in the Anti-PD1 group, and the concentration of ALT in blood increased in the RAPO group (Table 7).

**[Table 7] Serum AST and ALT levels**

| Group | AST (U/L) | ALT (U/L) |
|---|---|---|
| TC | 101.42±9.94 | 16.75±1.38 |
| RAPO | 103.38±9.40 | 20.60±1.08 |
| Anti-PD1 | 94.9512.51 | 18.6010.75 |
| Anti-PD1 + RAPO | 113.40±4.93 | 18.60±0.75 |
| P-value | 0.8031 | 0.7702 |

| | | |
|---|---|---|
| Note) Values were expressed as mean ± SEM. n = 4-5 per group. | | |

### (2) Renal function index (BUN, Creatinine)

The concentrations of serum BUN and creatine, which can be used to determine kidney functions, are shown in Table 8 and FIG. 4, respectively. BUN and blood creatine concentrations, which are generally used to diagnose acute renal failure, were measured in four groups. The result showed that there was no significant difference between the four groups in both indicators, but the BUN concentration decreased in the RAPO group and Anti-PD1 + RAPO group ingesting *Bifidobacterium* (Table 8).

**[Table 8] Serum BUN and creatine levels**

| Group | BUN (mg/dl) | Creatinine (mg/dl) |
|---|---|---|
| TC | 15.74±1.05 | 0.40±0.00 |
| RAPO | 14.73±1.63 | 0.40±0.00 |
| Anti-PD1 | 15.33±0.15 | 0.40±0.00 |
| Anti-PD1 + RAPO | 14.58±0.64 | 0.40±0.00 |
| P-value | 0.8168 | 0.5272 |

| | | |
|---|---|---|
| Note) Values were expressed as mean ± SEM. n = 4-5 per group. | | |

### (3) Fatigue-related index (LDH)

The blood LDH concentration, which is a fatigue-related index, is shown in FIG. 5. The blood LDH concentrations of the four groups were measured. The results showed that the Anti-PD1 group exhibited an increased blood LDH concentration and the blood LDH concentration was decreased by *Bifidobacterium* administration along with anti-PD1 treatment (Table 9).

**[Table 9] Serum LDH concentration**

| Group | LDH (U/L) |
|---|---|
| TC | 1234.20±126.12 |
| RAPO | 1291.80±128.71 |
| Anti-PD1 | 1347.75±20.93 |
| Anti-PD1 + RAPO | 1186.40±154.84 |
| P-value | 0.6482 |

| | |
|---|---|
| Note) Values were expressed as mean ± SEM. n = 4-5 per group. | |

### D. Observation of PDL1 expression in tumor tissues by immunohistochemistry (IHC) staining

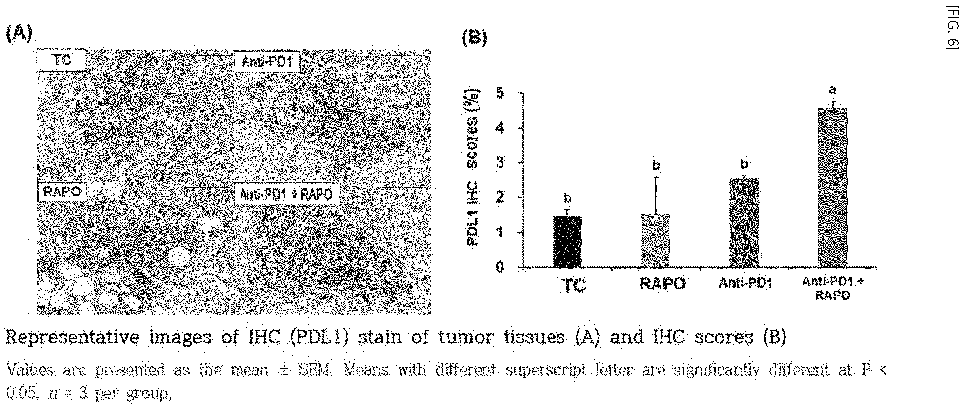

Anti-PD1 used in the preliminary experiment, among the immune checkpoint inhibitors, is known to increase the anticancer effect of the anti-PD1 immune checkpoint inhibitor as the expression of PD-L1 in the tumor microenvironment increases (Eno J. Immunotherapy through the years. J Adv Pract Oncol. 2017;8(7):747-753.). The expression of PDL1 in tumor tissue, which is a biomarker that can be used to predict the effect of anti-PD1, was detected through IHC staining. The result showed that the expression of PD-L1 in the tumor tissue of the Anti-PD1 + RAPO group significantly increased compared to the other groups (P=0.0120) (FIG. 6).

In addition, RT-qPCR was performed in tumor tissue to determine PDL1 mRNA expression. PDL1 gene expression in the Anti-PD1 + RAPO group was significantly increased compared to the other groups (P=0.0181) (FIG. 7).

### E. Flow cytometry of spleen and tumor tissue

In order to verify the effect of the combination therapy of immune checkpoint inhibitors and probiotics on the expression of immune cells related to chemotherapy, cells were extracted from spleen and cancer tissue and flow cytometry was performed thereon. The results are as follows.

### (1) Results of flow cytometry of spleen tissue

There was no significant difference between the groups in CD4+ CD3+ T cells and CD8+ CD3+ T cells in the spleen tissue, whereas the ratio of CD8/CD4 T cells was higher in the Anti-PD1 + RAPO group than in the Anti-PD1 group (C of FIG. 8).

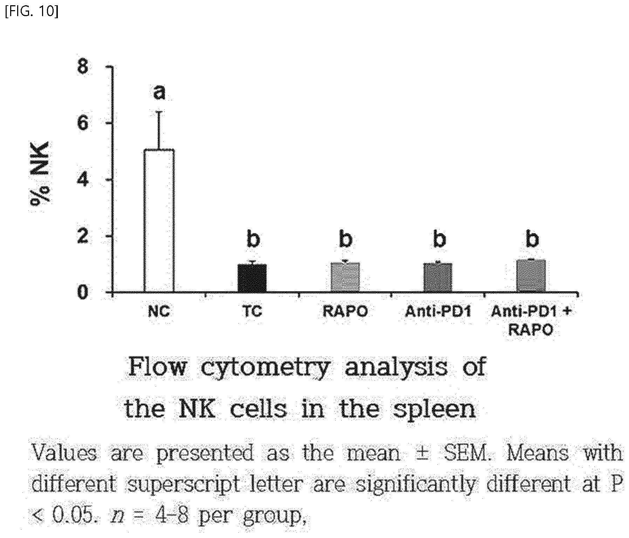

Natural killer T cells (NKT cells) in spleen cells were observed. The result showed that the Anti-PD1 + RAPO group exhibited a significant increase in NKT cells compared to the RAPO and Anti-PD1 groups (P<.0001) (FIG. 9). All four groups inoculated with tumors exhibited a significant decrease in natural killer cells (NK cells) compared to the NC group (P=0.0063) (FIG. 10).

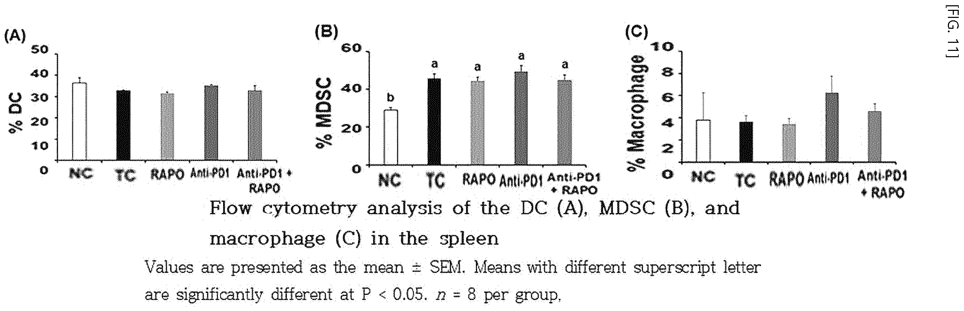

FIG. 11 shows the dendritic cells (DC), myeloid-derived suppressor cells (MDSC), and macrophages in the spleen tissue. There was no significant difference in DCs and macrophages between all groups and that MDSC significantly increased in the TC, RAPO, Anti-PD1 and Anti-PD1 + RAPO groups inoculated with tumors, compared to the NC group (P<.0001) (B of FIG. 11).

### (2) Results of flow cytometry of tumor tissue

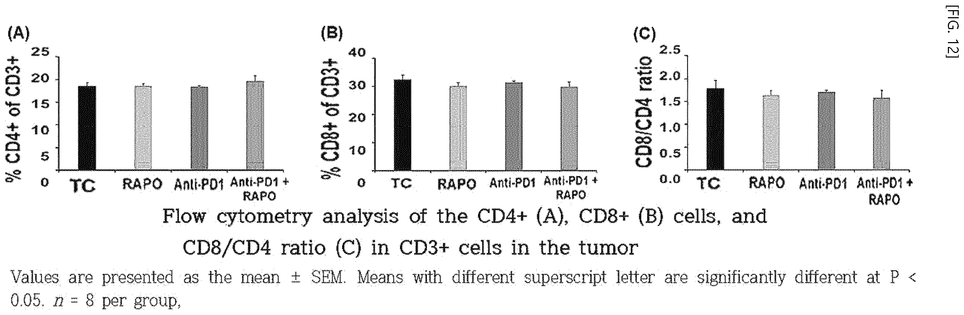

There was no significant difference between the groups in CD4+CD3+ T cells, CD8+CD3+ T cells, and CD8/CD4 ratio in tumor tissue (FIG. 12).

NK cells in the tumor tissue were observed. The result showed that the RAPO group exhibited a significant increase in NK cells compared to the TC and Anti-PD1 groups and the Anti-PD1 + RAPO group exhibited a slight increase in NK cells compared to the Anti-PD1 group (P=0.131) (FIG. 13).

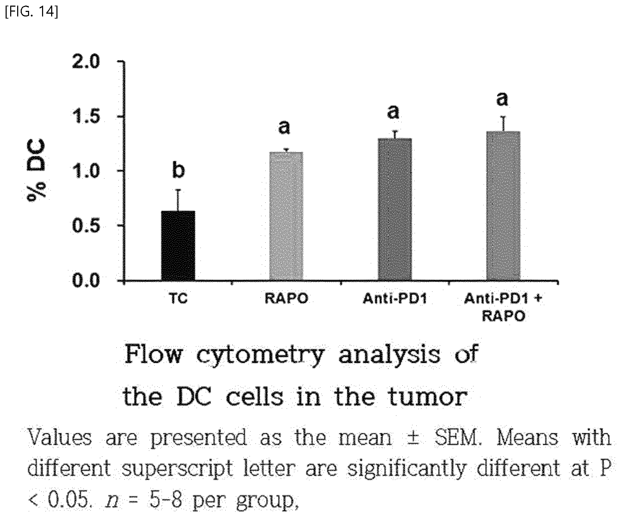

DCs in tumor tissues were significantly increased in the RAPO, Anti-PD1, and Anti-PD1 + RAPO groups compared to the TC group (P=0.0007) (FIG. 14).

FIG. 15 shows the M1 macrophages (anti-tumor) and M2 macrophages (pro-tumor) in the tumor tissue. M1 macrophages increased in the RAPO group, although there was no significant difference between the groups. The M2 macrophages significantly decreased in the Anti-PD1 + RAPO group compared to the TC and Anti-PD1 groups (P=0.0249) (B of FIG. 15). In addition, the M2/M1 macrophage ratio significantly decreased in the RAPO group compared to the TC and Anti-PD1 groups, the M2/M1 macrophage ratio significantly decreased in the Anti-PD1 + RAPO group compared to the TC group (P=0.0105), and the M2/M1 macrophage ratio significantly decreased in the Anti-PD1 + RAPO group compared to the Anti-PD1 group (P=0.0075) (C of FIG. 15).

### F. Results of gene expression measurement related to anti-cancer enhancement effect in tumor tissue

### (1) Measurement of tumor associated macrophages (TAM) and immune-related gene expression

mRNA expression in tumor tissue of interferon gamma (IFNγ), tumor necrosis factor alpha (TNFα), and C-X-C motif chemokine lignad 9 (CXCL9), among cytokines known as markers of M1 acting as an anti-tumor in the tumor microenvironment, was measured using RT-qPCR. The results are shown in FIGS. 16 to 18.

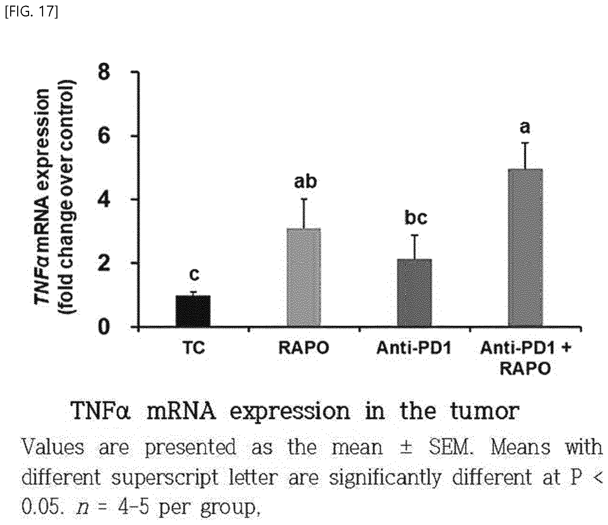

IFNγ mRNA expression in tumor tissues significantly increased in the Anti-PD1 + RAPO group (P=0.0223) (FIG. 16), TNFα gene expression significantly increased in the Anti-PD1 + RAPO group compared to the TC group and Anti-PD1 group, and TNFα gene expression significantly increased in the RAPO group compared to the TC group (P=0.0078) (FIG. 17).

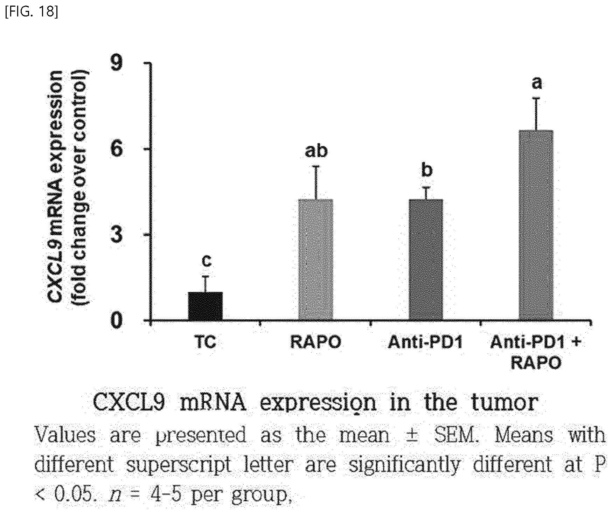

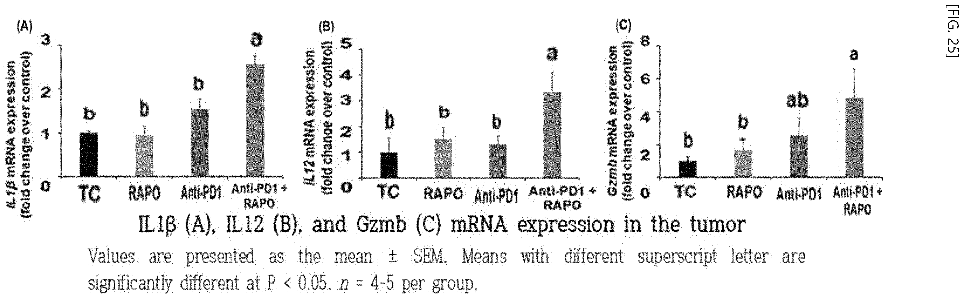

The gene expression of CXCL9 in tumor tissues significantly increased in the Anti-PD1 + RAPO group compared to the TC and Anti-PD1 groups (P=0.0028) (FIG. 18). In addition, the gene expression of interleukin 1β (IL1β) and interleukin 12 (IL12), known as cytokines secreted by M1 macrophages, in the tumor tissue, was detected. The result showed that the Anti-PD1 + RAPO group exhibited a significant increase in the gene expression compared to the other groups (IL1β, P=0.0002; IL12, P=0.0330) (A of FIG. 25 and B of FIG. 25) .

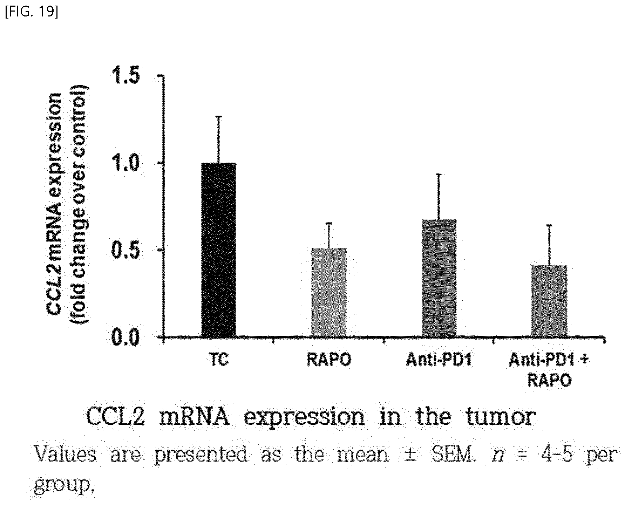

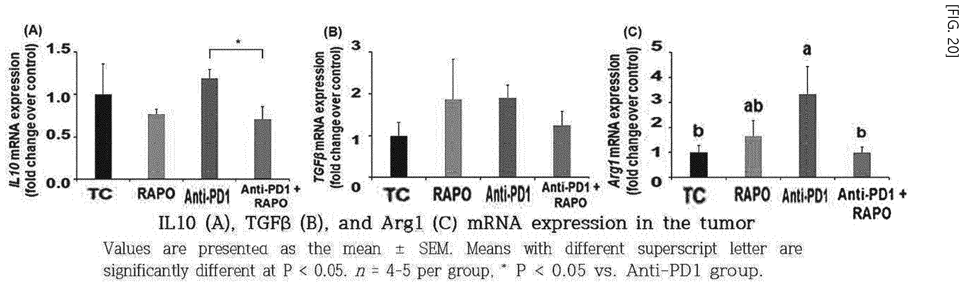

The mRNA expression levels in cancer tissues of C-C motif chemokine ligand 2 (CCL2), which is known as a marker of M2 acting as a pro-tumor and promotes the recruitment of monocytes and myeloid-derived suppressor cells (MDSCs) to increase tumor growth and metastasis, and interleukin 10 (IL10), arginase 1 (Arg1) and transforming growth factor β (TGFβ), among the cytokines known to be secreted by M2, were detected through RT-qPCR. The results are shown in FIGS. 19 and 20. The results showed that there was no significant difference between the groups in the gene expression of CCL2, whereas the gene expression decreased in the Anti-PD1 + RAPO group (FIG. 19).

The RAPO and Anti-PD1 + RAPO groups exhibited decreases in IL10 mRNA expression in tumor tissues compared to the TC and Anti-PD1 groups. In particular, the Anti-PD1 + RAPO group had a significant decrease in IL10 gene expression compared to the Anti-PD1 group (P=0.0394) (A of FIG. 20). When the gene expression of TGFβ in the tumor tissue was observed, there was no significant difference between the groups, but the Anti-PD1 + RAPO group exhibited a slight decrease in expression of TGFβ genes in the tumor tissue compared to the RAPO and the Anti-PD1 groups (B of FIG. 20). In addition, the expression of Arg1 mRNA in tumor tissues significantly decreased in the TC and Anti-PD1 + RAPO groups compared to the Anti-PD1 group (P=0.0445) (C of FIG. 20).

### (2) Measurement of immunogenic cell death (ICD)-related gene expression

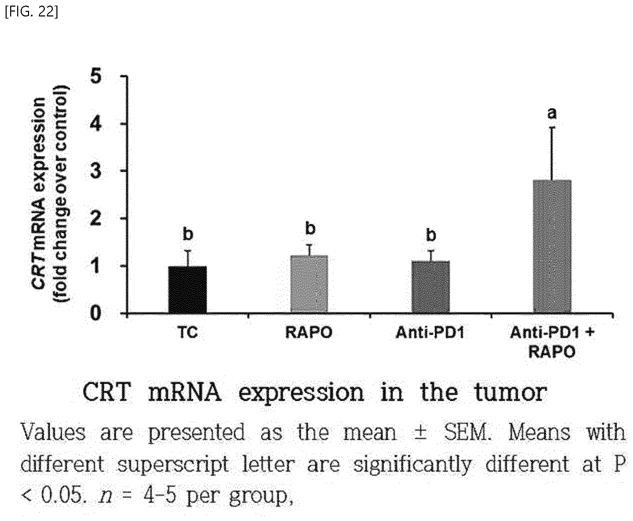

Damage-associated molecular patterns (DAMPs) secreted from dead cancer cells in the tumor microenvironment are known to induce maturation of dendritic cells (DCs) and thereby to enhance anticancer effects. Expression of high mobility group B1 (HMGB1) and calreticulin (CRT) genes among these DAMPs were observed in tumor tissues and the results are shown in FIGS. 21 and 22. The gene expression of HMGB1 in the tumor tissues of the Anti-PD1 + RAPO group significantly increased compared to the TC and Anti-PD1 groups (P=0.0271) (FIG. 21). In addition, CRT mRNA expression also exhibited a significant increase in the Anti-PD1 + RAPO group (P=0.0345) (FIG. 22).

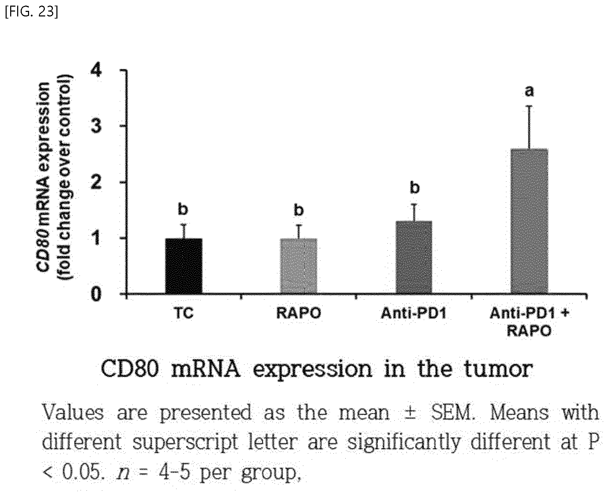

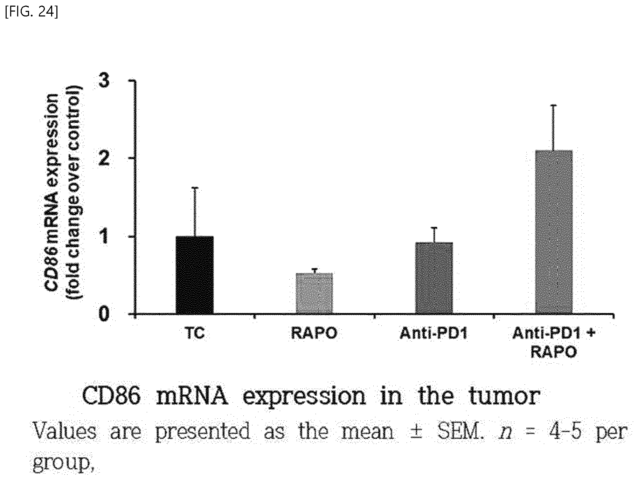

FIGS. 23 and 24 show the mRNA expression in tumor tissue of CD80 and CD86, known as DC markers activated by ICD. The CD80 gene expression in the tumor tissue significantly increased in the Anti-PD1 + RAPO group compared to the other groups (P=0.0225) (FIG. 23), and there was no significant difference between the groups in CD86 gene expression, but the Anti-PD1 + RAPO group exhibited an increase in CD80 gene expression compared to other groups (FIG. 24).

The gene expression of IL1β, interleukin 12 (IL12), and granzyme B (Gzmb), secreted by immune cells enhanced by ICD to kill cancer cells, among the cytokines known to be secreted by matured and activated DCs, was detected in tumor tissues. The results are shown in FIG. 25. The gene expression of IL1β in the tumor tissue significantly increased in the Anti-PD1 + RAPO group compared to the other groups (P=0.0002) (A of FIG. 25). IL12 mRNA expression in the tumor tissue also significantly increased in the Anti-PD1 + RAPO group (P=0.0330) (B of FIG. 25). The gene expression of Gzmb in tumor tissue significantly increased in the Anti-PD1 + RAPO group compared to the TC and RAPO groups, and increased slightly compared to the Anti-PD1 group (P=0.0416) (C of FIG. 25). In addition, the gene expression of IFNγ, which is one of the cytokines secreted due to an increase in ICD, also significantly increased in the Anti-PD1 + RAPO group (P=0.0223) (FIG. 16).

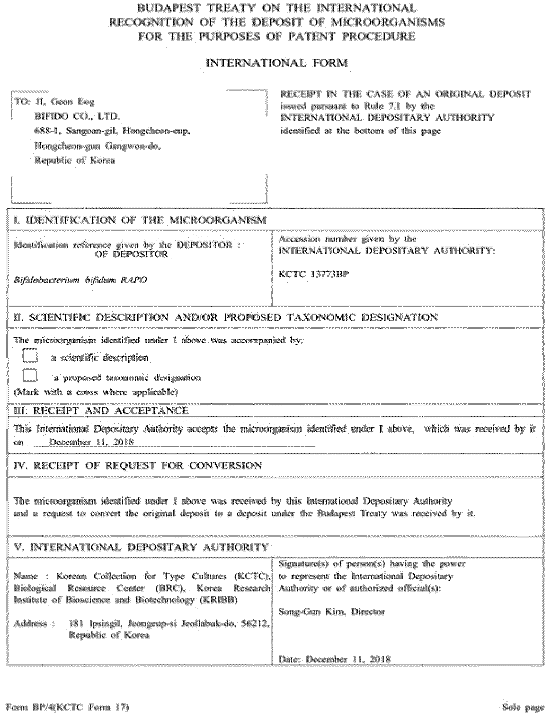

## Claims

1. A pharmaceutical composition for preventing or treating cancer comprising *Bifidobacterium longum* RAPO (KCTC13773BP).

2. The pharmaceutical composition according to claim 1, wherein the *Bifidobacterium longum* RAPO (KCTC13773BP) exhibits an immune-enhancing effect as well as anticancer activity.

3. The pharmaceutical composition according to claim 1, wherein the cancer comprises at least one selected from the group consisting of melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, hemangiosarcoma, mast cell tumor, leukemia, lymphoma, liver cancer, lung cancer, pancreatic cancer, stomach cancer, kidney cancer, colorectal cancer, hematopoietic tumor, neuroblastoma, epidermal carcinoma, and metastatic cancer thereof.

4. A pharmaceutical composition for preventing or treating cancer comprising *Bifidobacterium longum* RAPO (KCTC13773BP) and further comprising an immuno-anticancer agent.

5. The pharmaceutical composition according to claim 4, wherein the immuno-anticancer agent comprises at least one selected from the group consisting of anti-PD1, anti-PDL1, anti-CTLA, anti-Tim3 and anti-LAG3.

6. The pharmaceutical composition according to claim 4, wherein the *Bifidobacterium longum* RAPO (KCTC13773BP) strain and the immuno-anticancer agent are administered simultaneously in one formulation, or administered simultaneously or sequentially in separate formulations.

7. The pharmaceutical composition according to claim 4, wherein the *Bifidobacterium longum* RAPO (KCTC13773BP) reduces side effects of the immuno-anticancer agent.

8. A food composition for preventing or ameliorating cancer comprising *Bifidobacterium longum* RAPO (KCTC13773BP).

9. An animal feed composition for preventing or ameliorating cancer comprising *Bifidobacterium longum* RAPO (KCTC13773BP) .
